# EUROPEAN PATENT APPLICATION

(11) **EP 4 151 673 A1**
(43) Date of publication of application: **22.03.2023**
(21) Application number: 20935963.7
(22) Date of filing: 12.05.2020
(51) Int. Cl.: C08J 3/00, C08L 67/00, A61L 27/50

(54) **METHOD FOR PURIFYING BIODEGRADABLE THERMOPLASTIC POLYMER PARTICLES FOR MEDICAL AND/OR PHARMACEUTICAL USE**

(71) Applicant: Laboratorios Farmacéuticos Rovi, S.A., 28037 Madrid (ES)
(72) Inventor: GARCIA AMO, María, 28037 Madrid (ES); GUTIERRO ADURIZ, Ibon, 28037 Madrid (ES); CEBADERA MIRANDA, Elena, 28037 Madrid (ES)
(74) Representative: Balder IP Law, S.L.
(86) International application number: PCT/ES2020/070306
(87) International publication number: WO 2021/229111

(57) **Abstract**

Process for the purification of biodegradable thermoplastic polymeric particles for medical and/or pharmaceutical use without using organic solvents, as well as the particles obtained thereby, and the use of the polymeric particles obtained by this process for manufacturing medicaments for parenteral administration and/or implantable medical devices.

## Description

Process for the purification of biodegradable thermoplastic polymeric particles for medical and/or pharmaceutical use.

### Field of the Invention

The present invention falls within the field of the purification of polymeric particles obtained from biodegradable thermoplastic polymers for use thereof in the medical, pharmaceutical and/or healthcare fields. In particular, the process and the particles object of the present invention present the technical advantage of being obtained in an apyrogenic and low microbial load environment. More particularly, it concerns a process for the purification of polymeric particles in the absence of solvents, preferably consisting of copolymers of lactic acid, glycolic acid and caprolactone. The present invention also refers to the particles themselves, to the use of these particles in the healthcare field, such as for obtaining biodegradable medical devices (catheters, sutures, staples, surgical clips, implants) as well as in the formulation of implantable drugs (intramuscular or subcutaneous).

### Background Art

Synthetic biodegradable thermoplastic polymers have been used for years for medical applications, and their use is becoming increasingly widespread. Most of these biodegradable polymers are solid thermoplastic materials based on polymers and copolymers of lactic and glycolic acids and caprolactone. The administration of these biodegradable devices requires in some cases the use of a surgical incision to place the solid material at the incision site. In other variants, the biodegradable polymer is dissolved in an organic solvent, and the liquid formulation is administered using for example a syringe and needle, resulting in a solid implant at the site of deposition of the formulation by precipitation of the polymer upon contact with body fluids. One of the applications of these formulations constituted by synthetic biodegradable thermoplastic polymers is the addition of an active component to the formulation, giving rise in this case to active polymer implants from where the release of the active component is controlled by the biodegradation of the biodegradable polymer. Other applications are the formation of biodegradable catheters, sutures, staples or surgical clips as support material in medical applications.

As these are polymers for healthcare applications, the European and United States guidelines set very strict requirements for quality assurance (such as pressure, temperature and humidity control) which, logically, apply to pharmaceutical products [PDA J. Pharm. Sci and Tech. 2015, 69, 123, 139 (draft) PDA J. Pharm. Sci and Tech. 2018]. Additionally, in the case of parenteral formulations that are difficult to inspect visually, such as a parenteral formulation consisting of an active ingredient and a polymer, the guidelines require an ancillary test to verify that the product is essentially free of particulate matter. This additional test - specified in the chapters of the United States guidelines published by the United States Pharmacopoeia [USP 1790 Visual Inspection of Injections and USP 790 Visual Particles in Injections] - stipulates a visual inspection of a representative sample of the batch size, after dissolving them in a suitable solvent. In the visual inspection, no visible particles should be observed in the selected samples. According to the literature published to date, particles larger than 100 - 150 µm are considered visible.

The importance of early detection of these visible particles before they are inserted or implanted inside the human body is of utmost importance, since parenteral administration of inert particles can give rise to four pathogenic mechanisms: (i) local or systemic infections and inflammation caused by the presence of microorganisms or endotoxins; (ii) inflammatory response caused by the particle or derivatives thereof, which may result in tissue injury; (iii) allergic or anaphylactic reactions; and (iv) tissue damage caused by occlusion of blood capillaries [PDA J. Pharm. Sci and Tech. 2015, 69, 123, 139 (draft) PDA J. Pharm. Sci and Tech. 2018]. For this reason, in order to release such medical or pharmaceutical products to market with appropriate quality assurance, it is necessary to develop a process that allows the removal of such particles with size greater than 100 - 150 µm.

In this sense, the most common sources of particles in the manufacture of a parenteral drug or an implantable medical device is the process of obtaining them, since the use of raw materials, packaging materials, equipment, work environment and personnel involved in the entire manufacturing process are of significant importance.

For this reason, the inventors of the present invention have developed an alternative polymeric particle purification process to eliminate the presence of visible extrinsic particles of size greater than 100 - 150 µm in the pharmaceutical product or in the final medical device, "extrinsic" being understood as those particles of a nature different from that of the polymeric starting material, for example, any amorphous or defined particle of polymeric origin, such as fibres, granules, pellets, agglomerates, aggregates, among others. In particular, extrinsic particles can be organic, such as natural cellulose fibers, or inorganic, such as metals.

With regard to the prior art, it should be noted that there are well known processes for obtaining and polymerizing thermoplastic polymers, which, by way of illustration, consist of the following general steps: (i) polymerization reaction, (ii) removal of residual monomers, (iii) extrusion, (iv) cooling and molding, and (v) final conditioning. The final molding of the polymer can take many forms: pellets, catheters, spheres, particles, etc. However, none of the various prior art documents allow obtaining a sterile polymer.

In this regard, Japanese patent JP2012025968 in the name of NIPPON CATALYTIC CHEM IND describes a pelletizing process based on obtaining a thermoplastic acrylic resin. To do so, NIPPON CATALYTIC CHEM IND performs the polymerization reaction from which the thermoplastic acrylic resin is obtained. Then said resin is extruded using a temperature in the range of 220 to 300 °C. After extrusion, the resin is filtered through a metal filter with a pore size of 10 µm to remove contaminating particles with a particle size of 20 µm. Once the resin has been filtered, it is cooled with water at a temperature range of 30 to 80°C. Finally, it is pelletized to obtain a pellet of 4 mm in size. A substantial difference with the present invention consists in the cooling method, which in the case of the Japanese patent is carried out by water, but in the present invention is carried out by air instead of water due to the nature and physico-chemical properties of the biodegradable thermoplastic polymeric particles used, due to which sterile compressed air or inert gases are used. Another difference of the present invention with the Japanese patent is the absence in the process of the invention of the polymerization stage. This stage is essential in the Japanese patent, since it obtains the thermoplastic acrylic resin with essential features for the invention, from which it obtains the pellets of interest. Instead, in the present invention this stage is not present since the thermoplastic biodegradable polymeric particles are introduced directly in the extruder, independently of their origin.

Another close document of the prior art is the Chinese utility model CN206796507 filed by CHONGQING YOUHE NEW MAT CO LTD, which describes a device for extruding plastic comprising two extruders, each with a filter, as well as a cooling device connected to the second extruder. The device also includes a pelletizer. An important difference of the present invention with the Chinese utility model relates to the cooling by means of a water tank carried out by the Chinese utility model, as opposed to the air cooling carried out in the present invention. Furthermore, the Chinese utility model performs a double extrusion and filtering, whereas the present invention obtains the desired results with a single extrusion and filtering.

In addition, US patent application US5439688 in the name of DEBIO RECHERCHE PHARMACEUTIQUE S.A. describes a process for the preparation of a pharmaceutical composition in the form of microparticles. To do this, a polymer is mixed with a pharmaceutical salt. The resulting mixture is compressed and heated (maximum temperature 90°C) before it enters the extruder. In the extruder the mixture remains at a temperature between 90 and 100°C, and after its extrusion it is necessary to cool the mixture obtained by thermal transfer to a sterile cooled gas or air. Finally, the product is crushed by cryogenic grinding (0°C or even -30°C). This patent application has many differences with the present invention since at no time does it take into account the importance of the purification of the polymer to separate particles larger than 100 µm, since it is most likely intended for oral administration and does not require a degree of sterility as demanding as that of the present invention. Furthermore, an important feature of the present invention compared to this US patent lies in the filtering of the extruded product for the removal of contaminating particles or impurities thus purifying it, whereas in this patent application there is no filtration at any stage for the removal of impurities.

US Patent US09/212986 filed by AVENTIS PHARMACEUTICALS Inc. describes a method for producing a pharmaceutical composition based on a melt extrusion method for forming microparticles. The steps carried out to obtain the microparticles consist of mixing the polymer with a pharmaceutical salt to form a dry mixture, extruding this mixture to obtain a homogeneous mixture in the form of a strand, cooling the mixture and pelletizing said strand, and pulverizing the pellets to form microparticles with sizes from 10 to 200µm. This patent uses the spray or wet spray technique to obtain the final formulation. Again, this invention does not take into account the removal of impurities from the polymeric sample because the final aim is to that the final formulation contains the active principle in combination with the polymer in the form of a polymeric matrix.

On the other hand, international patent application WO2015/028060 A1 filed by EVONIK INDUSTRIES AG has been found, which describes a process for preparing an absorbable polyester in powder form obtained by a dissolution-precipitation process using organic solvents, with a specific density lower than that of the starting polymer. The substantial difference of the present invention with the international application of EVONIK INDUSTRIES AG consists in the advantage of not using solvents during the polyester preparation process. The substantial advantage posed by the present invention consists in the fact that, since no organic solvent is used, the physico-chemical properties of the purified polymeric particles do not vary with respect to those of the starting polymer. In contrast, the EVONIK patent describes a solvent purification process where the physico-chemical properties (such as the specific gravity of the purified product) vary from those of the starting polymer. In fact, a redensification step is described in the process to increase the density of the resulting product, but the density value of the starting polymer is not reached in any case. Another feature of the application by EVONIK is that it performs a filtration for a different reason than in the present invention, since, in the former case, the filtration is performed to remove the solvents used during the process, whereas in the present invention the filtration stage is performed to remove impurities or solid contaminating particles having a particle size of 100µm or more. Particles which are attached to their commercial polymeric particles and which render the product non-viable for the end products claimed in the present invention (injectable drugs and implantable medical devices). Therefore, one of the differences of the present invention is that it provides a simple method of reprocessing polymeric particles so as to remove visible extrinsic particles with a particle size of at least 100µm. This clearly innovative process consists in the selection of a sample of particles to be introduced directly into an extruder (without any previous preparation and without using solvents in the whole process), presenting the technical advantage that, due to the control and definition of the technical features of the process (dry extrusion and cooling and no use of organic solvents), it does not alter the initial physicochemical characteristics of the selected particles.

Chinese patent application CN105111423 A by PENG CHUNHAI describes the manufacture of a water-based polyester by condensation and uses organic solvents in its process. To obtain the polymer, the steps of (a) esterification, (b) filtration, (c) condensation polymerization reaction, and (d) extrusion of the mixture are followed. In the PENG CHUNHAI document, the filtration is prior to polymerization in order to eliminate monomers which are not of interest in the mixture of esters to be polymerized; in the present invention, however, the filtration stage is subsequent to the extrusion of the polymeric particles because the aim is to eliminate visible extrinsic particles larger than 100µm. Moreover, the Chinese patent application uses solvents during its procedure, whereas, on the contrary, the purification process described in the present invention is carried out dry, that is to say, with the absence of both organic solvents and water.

In addition, there are several patent applications describing different types of filtration devices for thermoplastic polymer extrusion processes, among which those mentioned below stand out.

Patent application WO2017/163180 A1 by C M PRODUZIONE S.R.L. describes a device for the continuous filtering of molten plastic materials coming from the plastic waste recycling industry and having a high presence of inorganic impurities. In this case the filters are used to remove inorganic products from the extrusion mixture, mainly heavy metals. One of the differences of this international patent application with the present invention is based on the substances used for extrusion and filtering: in the present invention millimetre-sized biodegradable thermoplastic polymeric particles are used, whereas, in the international patent application, the substances used are large-scale materials. Moreover, the present invention demands a meticulous control of the conditions required for the extrusion and filtering of the particles so that these do not lose their physico-chemical properties and the sterility of the process is guaranteed, whereas the international patent application does not describe during the document the conditions under which the process is carried out, so that it is unlikely that it could solve the needs addressed in the present invention.

Patent application WO01/47687 A2 by UNION CARBIDE CHEMICAL & PLASTICS TECHNOLOGY CORPORATION relates to a filtering device for molten polypropylene and ethylene-propylene copolymers, wherein the filter is intended to remove residues and aggregates from the polymeric materials. This international patent application describes a filter and a method for filtering polymer melts, and the described method consists of several steps: melting of the polymer, filtering of the polymer melt, extrusion to shape it, and cooling. This international patent application presents the steps of filtration and extrusion, also used in the present invention but in the reverse order, since in the international application extrusion is used to shape the copolymer obtained and filtration is used to eliminate the monomers of the starting polymers whose presence is not desired in the final copolymer. This is because what is pursued with the filtration and subsequent extrusion is to separate the large agglomerate particles from the molten copolymer in order to subsequently mix the filtered melt, whereas the present invention uses the filtration stage to remove contaminating particles or impurities of a size of 100 µm or larger which are found in the polymeric particles.

Chinese utility model CN202213190 by ANTEPU ENGINEERING PLASTICS SUZHOU CO LTD describes a filtration device with an attached vacuum pump for extrusion processes without interruptions due to filter blockage. In this case the filter is intended for the removal of residues from the melting process of highly polymeric materials. One of the main differences of this Chinese utility model with the present invention is that it is intended for the recycling industry of non-biodegradable plastics, whereas the present invention is intended for biodegradable polymeric particles of millimetre size.

On the other hand, there are numerous patents describing specific filters to be coupled to extruders, such as US patent US2008314815 A1 by GNEUSS STEPHAN or patent EP3088157 A2 by FIMIC s.r.l. However, the present invention protects a purification process for biodegradable thermoplastic polymeric biodegradable particles of millimeter size, very different from the molten plastic materials obtained from recycling for which such filters are used. Moreover, these applications of the prior art have as their object a filter with certain qualities far removed from those pursued by the present invention (cheap, offering optimum filtration in a short time and of a material having acceptable physical characteristics), whereas the present invention needs specific conditions to be controlled during the whole process so that the physical-chemical properties of the polymeric particles are not affected after the extrusion and filtration steps. Furthermore, the filter material is essential in the present invention since because the polymeric particles are for healthcare application, it must be a pharmaceutical grade filter, such as 316 steel or 304 steel, which is by no means cheap or versatile.

DR COLLIN GMBH has applied for the international patent WO2018069238 A1 describing a device and method for inspecting molten polymers obtained from plastic materials. The device consists of an extruder, a storage device, a pressure filter test device and an electronic control device coupled to the extruder. The subject matter of the present invention differs considerably from that of this international patent application. The present invention describes a method of purifying biodegradable thermoplastic polymeric thermoplastic particles; in contrast, this international patent application describes a method for inspecting molten polymers obtained from plastic materials. For the identification of the molten polymers, the international patent application has a code identification device, such as QR codes or barcodes. In addition, it has an extruder, a storage device where the identification device is located, a pressure filter test device and an electronic control device attached to the extruder. Both the devices used and the process carried out are clearly different from those of the present invention.

The filters described in the aforementioned patents are standard quality metal filters for the manufacture of non-implantable and non-parenteral industrial copolymers. The process claimed in the invention requires the use of filters of pharmaceutical quality, such as those made of 316 steel or 304 steel. Furthermore, the polymer melt filtration process described in these patents can be carried out in an environment with standard cleanliness requirements, whereas the filtration process disclosed in the present invention must be carried out in an environment with strict cleanliness requirements because it involves the purification of a polymer for sanitary use, which must comply with the various International Guidelines. On the other hand, for the cooling of water-insoluble thermoplastic materials such as those used for the industrial applications claimed in the patents described, to cool the polymer for its solidification the melt is passed through a container with cold water, whereas in the process described in the present invention, for the cooling of biodegradable polymers for parenteral application, cold air filtered by a 0.22 µm filter is used, since they are partially soluble in water.

As can be seen, no process such as that claimed in the present invention is currently described in the prior art for the purification of biodegradable thermoplastic polymers, without the use of solvents and without altering the physico-chemical properties of the polymer, for use in demanding healthcare applications, such as for obtaining implantable medical devices or parenteral pharmaceutical formulations (intramuscular, subcutaneous, intravenous, etc.) requiring a polymer purity level of at least 95%, preferably at least 97% and more preferably 99%, with no fibres of a nature other than that of the polymer.

Accordingly, the inventors of the present invention have developed an alternative polymeric particle purification process to eliminate the presence of particles larger than 100 - 150 µm in the pharmaceutical product or in the final medical device. For this purpose they have designed a process for the purification of biodegradable thermoplastic polymeric particles for sanitary use, in the absence of solvents, which provides a significant advantage since the physicochemical properties of these polymeric particles remain intact. Moreover, it is noteworthy that since the developed process does not use organic solvents at any stage of the process, the presence of residual solvents in the product is avoided (with the consequent subsequent manipulations necessary to eliminate them), and the physicochemical properties of the polymeric particles with respect to those of the starting polymer are kept intact, thereby avoiding the possible appearance of degradation substances.

A further advantage of the present process is that it is carried out under sterile conditions, by which is meant apyrogenic and low microbial load conditions. This is an essential requirement in parenteral administration products and implantable medical devices. In addition, the process developed by the inventors of the present invention makes it possible to remove all particles larger than 100 -150 µm. The particles removed by this process can be of organic nature such as natural cellulose fibers, inorganic such as metals, and even aggregates from the melting of the polymer itself.

Therefore, the purification process of the present invention allows obtaining polymeric particles essentially free of visible extrinsic particles, with a polymer purity level of at least 95%, preferably at least 97% and more preferably 99%, presenting the advantage preserving the physicochemical characteristics of the starting product (i.e. the same inherent viscosity, the same bulk density, the same moulding capacity, etc.) additionally allowing to obtain a customised particle size depending on the drug or medical device required to be obtained therefrom.

### Brief Description of the Figures

**Figure 1** shows a diagram representing the solidification and cooling process of non-crystalline and partially crystalline thermoplastic polymers with a specific volume change with temperature. Figure 1 shows that there is a decrease in specific volume with decreasing temperature that presents a change of slope at a characteristic temperature of the material, known as the glass transition temperature, Tg. Above this temperature the polymer has a viscous behavior (rubbery, elastic), and below it a brittle glass behavior.
**Figure 2** shows a photograph taken with a dry binocular microscope of a sample of Lactel^{®} (Durect^{®} PLGA) particles as received for the beginning of the process object of the present invention. This image shows the presence of cellulose fibres in the totality of the polymeric particles.
**Figure 3** shows a photograph taken with a binocular microscope of a sample of Lactel^{®} particles (Durect^{®} PLGA) subjected to dissolution in an acetone solution. This image shows the presence of extrinsic fibers or particles in the solution.
**Figure 4** shows an IR spectrophotometric image of the nature of the extrinsic particles obtained after dissolving Durect^{®} PLGA in suspension. From this spectrum it was determined that the extrinsic particles are mostly cellulose and polyester.
**Figure 5** shows particles obtained after the process of the present invention with a degree of purity of at least 95% with respect to the starting polymeric particles. As can be clearly seen there are no extrinsic particles.
**Figure 6** shows a dissolution in acetone of the particles obtained after the process of the present invention with a purity of at least 95% with respect to the starting polymeric particles. As can be clearly seen, no particles are visible because the dissolution is complete.
**Figure 7** shows microparticles obtained after carrying out the process of example 3. This image shows the total absence of extrinsic particles, presenting a degree of purity of at least 95% with respect to the starting polymeric particles. As can be clearly seen there are no extrinsic particles and the sample of microparticles is totally homogeneous.

### Description of the Invention

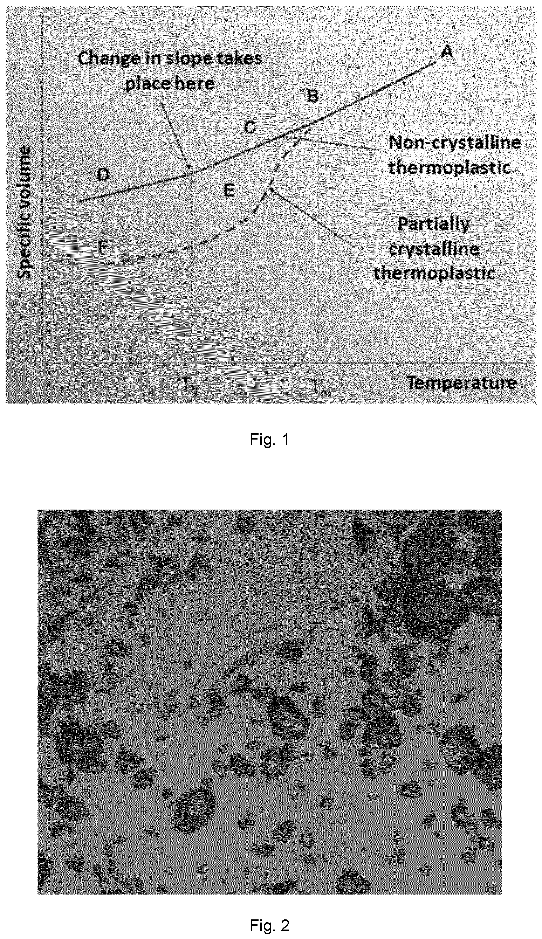

As used herein, unless otherwise specified the following terms should be understood to have the meanings indicated below:
"Biodegradable" refers to a material that can be degraded within the body, so that it is generally not disposed of intact.
"Biomaterial" includes all materials suitable for coming into contact with body tissues for specific therapeutic, diagnostic, or preventive purposes. These materials must be biocompatible.
"Biocompatible" refers to a material that does not cause any significant adverse response of the physiological environment upon interaction with body tissues and fluids, and must sometimes be biodegraded into non-toxic components, both chemically and physically, or by a combination of both.
"Particles" according to the present invention, refers to particulate systems of various shapes with a particle size ≥ 1 mm. For the purpose of the present invention, the term "particle" comprises any amorphous or defined particle of polymeric origin, such as granules, pellets, agglomerates, aggregates, among others. The particles are used as a final product or as an intermediate product in the manufacturing process.
"Extrinsic particles" according to the present invention, refers to visible particulate systems of a different nature from the polymeric starting materials, which are detectable when such polymeric materials are subjected to dissolution in a medium in which they are totally dissolved. That is to say, those particulate systems that are insoluble in a medium in which the polymeric starting material is totally soluble. This definition shall include any amorphous or defined particle of polymeric origin, such as fibers, granules, pellets, agglomerates, aggregates, among others.
"Granules" refers to formulations consisting of agglomerates of small particles or powders, which may be spherical or irregular in shape.
"Pellet" refers to a material which is compacted into the form of small spheres or cylinders by processes such as compaction, extrusion and/or spheronization (melting of the solid mass, wetting of the dry mass, extrusion of the wet or molten mass and rotation of the extrudate by spheronization and subsequent drying).
"Microparticles" are spherical or non-spherical particles with mean diameters between 100 and 150 µm, preferably a mean diameter of 125 µm. This group includes microcapsules, which are defined as vesicular systems in which the drug is confined in a cavity surrounded by a single (usually polymeric) membrane, and microspheres, which are matrix systems in the form of spherical particles between one and several tens of microns in size, without a distinction between shell and core, in which the drug is dissolved or dispersed within a matrix made up of support materials, generally biocompatible polymers with a wide range of release rates and degradation properties.
"Nanoparticles" are submicron (< 1 µm) particulate systems. According to the process used to prepare nanoparticles, nanocapsules or nanospheres can be obtained, these being the morphological equivalents of microcapsules and microspheres, respectively.
"Tg" or "glass transition temperature" refers to the temperature at which a thermodynamic pseudo-transition occurs in glassy materials. As can be seen in Figure 1, this is an intermediate temperature point between the molten state (Tm) and the rigid state of the material. Controlling Tg is important because when thermoplastics solidify from the liquid state they can form either a non-crystalline solid or a crystalline solid. A review of the solidification and slow cooling of non-crystalline or semi-crystalline thermoplastics shows that there is a decrease in specific volume with decreasing temperature that presents a change of slope at a temperature characteristic of the material known as the glass transition temperature, Tg, above which the polymer has a viscous behavior (rubbery, elastic) behavior and below which it has the behavior of brittle glass. This rapid decrease is due to the packing of the polymeric chains in the crystalline regions of the material, since the structure of the material is composed of crystalline regions immersed in an amorphous matrix of subcooled liquid, which below Tg passes to the glassy state, the structure then being formed by crystalline regions immersed in a glassy amorphous matrix.
"Tm" means melting temperature (Tm), which is the temperature at which the phase transition from solid to liquid or molten state occurs at normal atmospheric pressure.

For the purpose of the present invention, "dry" purification means a process of purification of polymeric particles in which neither water nor organic solvents are used in any of the steps. This aspect is essential in the present invention since it allows to obtain purified particles with the same structural properties as the starting ones, without altering the physicochemical properties thereof.

In the present description, "sterile conditions" means the environmental, equipment, facilities, room and working conditions that are suitable for obtaining an apyrogenic and low microbial load product; "apyrogenic" means a product that has a pyrogen load below 1 EU/mg (endotoxic units per milligram) and "low microbial load" means a product that has less than 300 CFU/mg (colony forming units per milligram).

With regard to the solution proposed by the inventors of the present invention, it is noteworthy that biodegradable plastic polymers (PLGA, PLA, etc.) are generally found in parenteral pharmaceutical preparations in the form of concentrated solutions of the polymer in organic solvents, and since, on the one hand, they have a strong tendency to form intermolecular esters (*e.g*. dimeric and polymeric lactic acid) and, on the other hand, they are strongly hygroscopic products, the degradation rate of the polymer in these formulations is higher than that of the dry polymer, which hinders purification on an industrial scale, making it a very complicated and laborious procedure.

In addition, to facilitate the formulation of the final drug or medical device, polymeric particles of a small size or of a particular size distribution are generally required which must be obtained for the final drug or medical device. These steps of processing the polymers to reduce particle size, for example by milling or grinding, contribute to the contamination of the product obtained, reducing its purity by up to 20% compared to that of the starting product.

For this reason, the researchers of the present invention have developed an alternative process of purification of biodegradable thermoplastic polymeric particles from that of the prior art, in dry state, which allows processing the starting polymer to customize it according to the rheological needs of the final product without altering the physicochemical properties of the starting product.

In this regard, a process for obtaining thermoplastic polymers comprised in the prior art with the aim of purifying them by the process claimed in the present invention is detailed below.

Polymerization reactions to obtain thermoplastic polymers (both homopolymers and copolymers) for parenteral use such as polymers of lactic acid (L,D,DL), glycolic acid and/or caprolactone polymers such as poly (L-lactic), poly (D-lactic), poly (DL-lactic), polyglycolic, poly ( 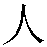 -caprolactone), copolymers of L-lactic/S-lactic, L-lactic/DL-lactic, L-lactic/Glycolic, DL-lactic/glycolic, or L-lactic/caprolactone acids, among others, can be carried out under a variety of temperature, time, and pressure conditions. These polymerization reactions are preferably carried out in the absence of oxygen.

On the other hand, there are polymers which are of interest also for the present invention such as: homopolymers or thermoplastic copolymers for medical use, non-parenteral such as PC (polycarbonate), PE (polyethylene), PEEK (polyether ether ketone), PEI (polyetherimide), PES (polyethersulfone), POM (polyoxymethylene), PP (polypropylene), PPS (polyphenylene sulfide), PPSU (polyphenylsulfone), PS (polystyrene), PSU (polysulfone), PTFE (polytetrafluoroethylene) and UHMWPE (ultrahigh molecular weight polyethylene), among others.

Generally, in the prior art, polymerization is carried out by incorporating the starting monomers to obtain the thermoplastic polymer in the reactor, and the reactor is then closed and pressurized, generally using inert gas, such as nitrogen, oxygen or hydrogen, among others. The reactor is then heated on a slow temperature gradient, between 0.1°C/min and 5.0°C/min, from room temperature. Once the monomers have partially melted, stirring begins and continues until the reactor has reached a temperature of at least 5°C above the melting temperature (Tm) of the monomers. At this time the stirring is stopped and the reactor is depressurized. The catalyst is then incorporated into the reactor, and the reactor is closed and pressurized with inert gas. Stirring is restarted and maintained for a sufficient time to obtain a homogeneous mixture of all the components. Once this homogeneous mixture is obtained, stirring is stopped and the reactor is depressurized. The initiating agent is incorporated into the reactor, and the reactor is closed and pressurized with inert gas. Stirring is restarted and maintained until the reactor mixture has reached a certain viscosity. At this time stirring is stopped, and both the temperature and the positive pressure of the inert gas are maintained for as long as is necessary for the polymerization to be completed, typically between 9 and 35 hours. Once the polymerization is completed, the residual monomers are removed. To do this, the temperature is maintained and a slight stirring and vacuum is applied to the molten mixture for the volatilization of the residual monomers. Finally, the polymer is extruded from the reactor. To do this, the temperature is maintained and the stirring is stopped, pressurizing with inert gas and opening a discharge valve so that the molten polymer flows from the reactor. At the outlet of the reactor the polymeric fluid is solidified by cooling with water until the polymer is solid, and it is reserved for subsequent use either in the form of particles, granules, pellets, etc.

This type of polymeric particles obtained by the polymerization process described above is marketed by the manufacturers with pharmaceutical quality. However, the researchers of the present invention have determined that, despite being of pharmaceutical quality, they do not have the degree of purity necessary to be used in parenteral systems (drugs or medical devices), so they have been forced to purify them by a dry reprocessing method, in order to keep the physicochemical properties of the starting particles intact but free of particles of compounds or substances foreign to the structure of the polymer of choice.

Thus, the inventors of the present invention have determined a dry purification method that makes it possible to obtain polymeric particles and/or microparticles of biocompatible biodegradable polymers with a purity level of at least 95%, preferably of at least 97% and more preferably of 99%, without fibers or particles of a different nature from that of the polymer of choice.

In view of the foregoing and according to a first aspect, the present invention protects a process for the dry purification of biodegradable thermoplastic polymeric particles for medical and/or pharmaceutical use comprising the following steps:
a) providing a particulate biodegradable thermoplastic polymer in a reactor,
b) extruding the polymer at a temperature of 4.5 to 5.5°C above the melting temperature (Tm) of the selected polymer and with a positive pressure greater than 0.1 bar in the presence of an inert gas,
c) filtering the polymer by means of at least one filtration step with at least one filter of pore size from 5 to 300 µm,
d) cooling the extruded polymer by means of a sterile air stream to a temperature below that of step b) until the polymer reaches a temperature of at least 4.5 to 5.5°C below the glass transition temperature (Tg), and
e) disintegrating the cooled polymer sample to obtain polymeric particles with a particle diameter ≥ 1 mm.

The cooling step is carried out by applying an air stream at a temperature below that of step b) such that the polymeric particles reach a temperature of at least 4.5 to 5.5°C below the glass transition temperature (Tg), since at this temperature an optimal thermodynamic pseudotransition occurs in vitreous materials (glasses, polymers and other amorphous inorganic materials). If this temperature were not controlled, the physicochemical properties of the selected polymeric particles would be altered in such a way that, above Tg, the polymeric particles decrease their density, hardness and rigidity, which conditions the subsequent processing of the material for its disintegration, particularly if they are parenteral or implantable systems. Preferably, cooling step (d) is carried out with sterile compressed air through a HEPA filter with pore size 0.22 µm or with sterile inert gas at a temperature below that of step b) until the polymer reaches a temperature of at least 4.5 to 5.5°C below the glass transition temperature (Tg).

Then, the solidified polymer is broken down into smaller fragments of several millimeters in diameter, which are dried by applying vacuum in a temperature range of 9 to 11°C to 34.5 to 5.5°C for an estimated time of 16 to 32 hours, to obtain polymeric particles with particle diameter ≥ 1 mm.

In another embodiment, a filter of pore size from 100 to 150 µm is used in filtering step (c). Preferably, the filter used in this filtering step is of pharmaceutical quality metal such as 304 stainless steel and 316 stainless steel.

In another embodiment, filtering step (c) is performed by means of more than one filter located in series, in parallel, in cascade, or in any combination thereof. Preferably, between step (b) and filtering step (c), an extruded polymer drive pump is incorporated in order to promote filtering.

According to another embodiment, the extrusion step (b) is carried out in a reactor with stirring by blade or paddle, turbine, propeller, anchor, spiral or worm screw. Preferably, the reactor is pressurized using an inert gas selected from the group consisting of nitrogen, argon, helium and compressed gas. In the extrusion process, once the polymeric particles have partially melted, stirring begins and continues until the reactor has reached a temperature of at least 4.5 to 5.5°C above the melting temperature (Tm) of the polymer. During the extrusion the temperature is maintained and the stirring is stopped, pressurizing with inert gas and opening a discharge valve so that the molten polymer flows from the reactor.

According to another embodiment, the process of the present invention, after the disintegration step (e), comprises a further drying step (f) and a further milling or sieving step (g) which is carried out by applying vacuum for a time of at least 10 hours at room temperature under sterile conditions, where particles with a diameter of ≤ 1 mm are obtained.

According to another embodiment, the disintegration step (e) is carried out by a blade system.

According to another embodiment, a method as described herein may comprise a further step (h) of sterilization, performed after step (e), (f) or (g) as described above, wherein the polymeric particles are subjected to a beta radiation dose equal to or higher than 25 kGy.

According to another embodiment, the process of the present invention is carried out in a sterile environment. In addition, before starting, all equipment is sterilized with nebulized or vaporized hydrogen peroxide or hydrogen peroxide mixture with peracetic acid.

Preferably, the polymeric particles to be purified are selected from the group consisting of particles of homopolymers and copolymers of lactic acid (L, D, DL), glycolic acid and/or ε-caprolactone such as poly(L-lactic), poly(D-lactic), poly (DL-lactic), polyglycolic, poly (ε-caprolactone), copolymers of L-lactic/S-lactic, L-lactic/DL-lactic, L-lactic/glycolic, DL-lactic/glycolic or L-lactic/ ε-caprolactone acids, PC, PE, PEEK, PEI, PES, POM, PP, PPS, PPSU, PS, PSU, PTFE or UHMWPE.

According to another embodiment, the present invention relates to a process for dry purification of biodegradable thermoplastic polymeric particles for medical and/or pharmaceutical use comprising the following additional steps:
f) drying the particles under vacuum for a time of at least 10 hours at room temperature, and
g) milling or sieving the particles until obtaining microparticles with an average diameter between 100 and 150 µm.

According to another embodiment, the microparticles are PLGA or PLA particles. Preferably, the microparticles are PLGA or PLA particles and have the following size distribution:
- D10: between 25 - 55 µm;
- D50: between 120 -170 µm;
- D90: between 300 - 375 µm.

The present invention also relates to a polymer particle obtained by the process of the present invention characterized in that it is sterile and therefore suitable for parenteral formulations or for manufacturing implantable medical devices.

Polymeric particles obtained by the process of the present invention are characterized by being essentially free of visible extrinsic particles, with a level of purity of the polymer of at least 95%, preferably of at least 97% and more preferably of 99%, having the advantage of preserving the physicochemical characteristics of the starting product (i.e., the same inherent viscosity, bulk density, molding capacity, etc.) further allowing to obtain a customized particle size depending on the drug product or medical device that is required to be obtained from them.

Generally, the properties of the polymeric particles obtained by the process of the present invention will depend on the properties of the starting polymers. In particular, the bulk density (ρ_{b}) and the tap or tapped density (ρₜ) of the polymeric particles obtained by the process of the present invention preferably correspond to the bulk density and the tapped density of the polymeric particles of the starting product.

By way of example, the bulk density (ρ_{b},) of the polymeric particles obtained by the method of the present invention may be, for example, in the range of 0.10 g/cm³ to 9.0 g/cm³ and the tapped density (ρₜ) thereof may be, for example, in the range of 0.13 to 12.0 g/cm³.

The bulk density (ρ_{b}) of the polymeric particles obtained by the process of the present invention and the tapped density (ρₜ) can be determined using methods and apparatuses known in the prior art. For example, as described in document WO2015/028060. In particular, method 1 indicated by the United States Pharmacopeial Convention (USP<616>) or method 1 indicated by *European Pharmacopoeia (Ph.Eur. 7.0*/*2010: 2.9.34)*.

Further, polymeric particles obtained by the process of the present invention may be characterized by the absence of residual solvent. In particular, since no solvent is used in the purification process as described herein, that is, it is a dry purification, the amount of residual solvent is typically 0.00%, calculated as the total amount of solvent per total weight of biodegradable thermoplastic polymer; preferably, the residual amount of solvent can be preferably 0.000% or even 0 ppm.

Residual amounts of solvents may be found only if the starting polymeric particles have been previously purified using some type of solvent. Preferably, the starting polymeric particles have not been previously purified, or have not been previously purified with a method using solvents. In particular, in a process as described herein, it is preferably characterized in that the biodegradable thermoplastic polymer provided in step a) has not been previously purified, or has not been previously purified with a method using solvents.

Residual solvents are determined by methods known in the art, e.g., as per ICH Q3C (R6) on impurities: Guideline for Residual Solvents of the European Medicines Agency (EMA/CHMP/ICH/82260/2006), published on 9 August 2019. In particular, the residual amount of specific solvents is below the limits indicated in this guide.

Generally, the polymeric particles obtained by the process of the present invention can be characterized by a residual solvent content (*e.g*. acetone) lower than 0.5%, in particular lower than 0.3%, more particularly lower than 0.1%, and more particularly lower than 0.05%, even lower than 0.01%, calculated as total amount of solvent per total weight of biodegradable thermoplastic polymer.

The absence of visible extrinsic particles can be easily determined by observing a sample of the particles under a microscope, for example using a binocular microscope, or using infrared (IR) spectrophotometry, which allows the presence of components of a chemistry different from that of biodegradable polymeric particles to be determined.

As described above for processes of the present invention, the polymeric particles obtained can be typically sterile, also referred to as apyrogenic and/or with low microbial load; "apyrogenic" being understood as a product having a pyrogenic load below 1 EU/mg (endotoxic units per milligram) and "low microbial load" as a product having less than 300 CFU/mg (colony-forming units per milligram), determined by methods known in the art, in particular in accordance with the United States Pharmacopeial Convention, respectively by USP <85> Bacterial Endotoxins Test and USP <61> Microbial Enumeration Tests.

In specific embodiments of a method as described herein, the obtained polymeric particles may have a microbial load of less than 100 CFU/g, and a pyrogenic load of less than 0.05 EU/mg. Such loads may be obtained, for example, after sterilizing the polymer by beta radiation at a dose which is equal to or higher than 25 kGy. Likewise, a method as described herein may comprise a further step (h) of sterilization, performed after step (e), (f) or (g) as described above, wherein the polymeric particles are subjected to a beta radiation dose which is equal to or higher than 25 kGy.

According to the foregoing description, in various aspects, the present invention also relates to biodegradable thermoplastic polymeric particles characterized by having an bulk density of 0.10 to 9.0 g/cm³, a tapped density of 0.13 to 12.0 g/cm³, an amount of residual solvent of 0.00%, in particular 0.000%, a pyrogenic load below 1 EU/mg and a microbial load below 300 CFU/mg and a size distribution
- D10: between 25 - 55µm;
- D50: between 120 -170µm;
- D90: between 300 - 375µm.

Particularly, what is described above relating to the method of the invention and to the particles obtained by said method also applies to the polymeric particles in this aspect of the invention.

According to a last aspect, the present invention relates to the use of the polymeric particles and/or microparticles for manufacturing parenteral administration medicaments and/or implantable medical devices.

Similarly, in related aspects, the invention relates to biodegradable thermoplastic particles for use as parenteral administration medicaments and/or implantable medical devices. Also, in related aspects, the invention also relates to a method of treating a disease comprising administering the polymeric particles and/or implanting the medical devices obtained with the polymeric particles.

Also, in related aspects, the invention relates to the manufacture of medical devices from the obtained biodegradable thermoplastic particles and to the devices obtained thereby.

### Examples

The following specific examples provided herein serve to illustrate the nature of the present invention. These examples are included for illustrative purposes only and are not to be construed as limitations to the invention claimed herein.

In such examples, PLGA-type (lactic or glycolic acid) and PLA-type (polylactic acid) thermoplastic polymers have been used.

The determination of bulk density (ρ_{b}) and tapped density (ρₜ) has been carried out according to method 1 indicated by United States Pharmacopeial Convention (USP<616>) or method 1 indicated by *European Pharmacopoeia (Ph.Eur. 7.0*/*2010: 2.9.34)* using the SOTAX tD1 equipment.

The determination of pyrogenic load (EU/mg, endotoxic units per milligram) and microbial load (CFU/mg, colony-forming units per milligram), has been carried out according to the United States Pharmacopeial Convention respectively by USP <85> *Bacterial Endotoxins Test* and USP <61> *Microbial Enumeration Tests.*

Example 1. Process for purification of PLGA thermoplastic polymer.

In this example, it is desired to purify 50/50 PLGA polymeric particles of the brand Durect^{®} (in particular Lactel DL-PLG (B6010-1): ester termination, intrinsic viscosity (IV) of 0.26-0.54 dL/g and bulk and tapped densities of the non-purified polymer of 0.64 and 0.84 g/cm³ respectively).

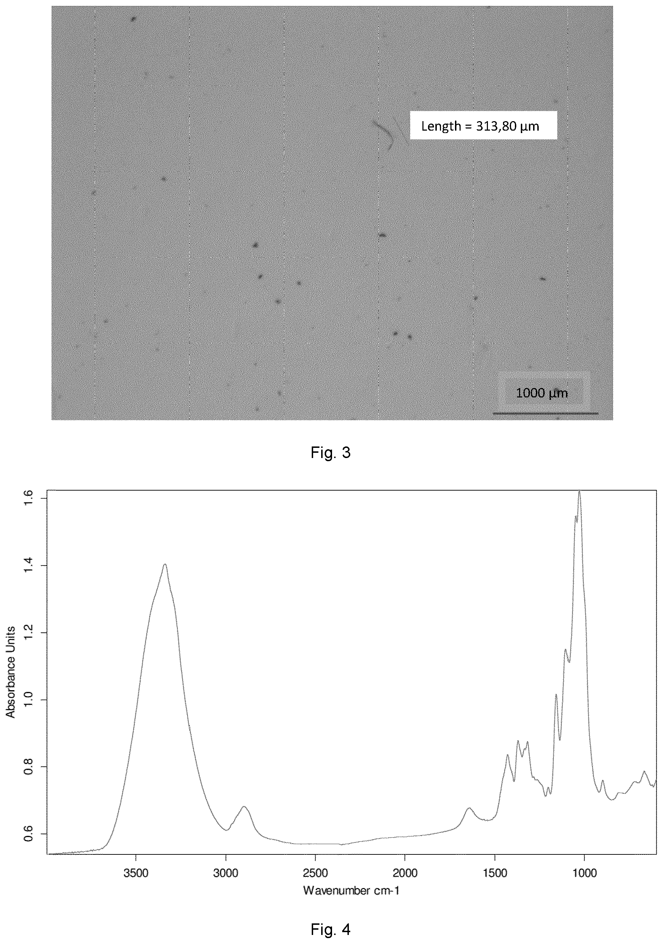

Before starting, a sample of the commercial polymeric particles to be purified is analyzed under a microscope to assess the degree of visible non-polymeric (extrinsic) particles to be removed. In this way, Figures 2 and 3 show an image taken with a binocular microscope where fibers larger than 100 µm with a morphology very different from the PLGA particles that are intended to be purified are seen. This image shows a clear indication that the acquired PLGA particles (of pharmaceutical quality) are not really suitable for parenteral use and therefore require an additional purification process.

The researchers, after observing the morphologically different particles, chose to analyse them using FTIR spectroscopy, finding that, for the most part, they are cellulose fibers (Figure 4). Therefore, the researchers proceeded with the dry purification process object of the present invention.

It should be noted that the purification process begins with an extrusion step performed within a reactor and that, before starting the purification process, all equipment and materials to be used must be clean and sterile. For this purpose, firstly either the entire equipment is sterilized with nebulized or vaporized hydrogen peroxide, or a mixture of hydrogen peroxide and peracetic acid, or the entire equipment is disinfected with disinfectants known in the art.

Additionally, in the case of injectable pharmaceutical quality products, it is required that both the work rooms and all the equipment are in sterile conditions.

To begin the purification process, the polymer in the form of particles is incorporated into the reactor. Once the polymeric particles are introduced into the reactor, the reactor is closed and pressurized with an inert gas such as nitrogen.

Then, the reactor is heated with a slow temperature gradient, 2°C/min ± 10%, from room temperature. When the particles have partially melted, stirring in the reactor is started with a few blades and continues until a temperature above the melting temperature (Tm) of the polymer is reached, i.e. a temperature in a range of 20-70°C ± 10%. Once these conditions are reached, the stirring is stopped and the reactor is depressurized. Maintaining the temperature, stirring is stopped and the reactor is pressurized with nitrogen. After this, the discharge valve is opened so that the molten PLGA flows from the reactor, through a filter with a pore size of 100 µm. This filtration removes contaminating particles that have not melted in the polymeric mass, obtaining a purified PLGA with a specific gravity identical to that of the non-purified polymer. In particular, obtaining particles of purified PLGA with bulk and tapped densities identical to those of the non-purified starting polymer.

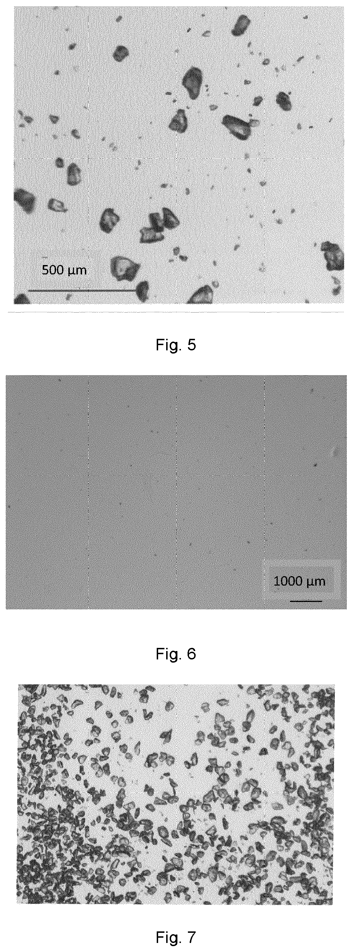

The polymeric mass is then cooled by means of a sterile air stream to a temperature of at least 4.5 to 5.5°C below Tg, and breaking down of the cooled polymer sample is subsequently started to obtain polymeric particles with a particle diameter ≥ 1 mm. Finally, a sample of the particles is taken after the purification process and they are observed again under a microscope and subsequently analyzed, observing how the cellulose fibers disappear from the initial commercial sample (Figures 5 and 6)

The bulk density of the particles obtained is 0.64 g/cm³ and their tapped density is 0.84 g/cm³, both coinciding with the density of the starting product.

In addition, due to the use of solvents in the purification process, the particles obtained do not contain a residual amount of solvent. The amount of residual solvents is below the detection limits and the total amount of residual solvents is 0.000%, since it is based on a polymer not previously purified with solvents and is therefore below 0.1%, the most restrictive limit for solvents of class 3 of the guideline: *ICH* Q3C *(R6) on impurities:* Guideline for Residual Solvents of the European Medicines Agency (EMA/CHMP/ICH/82260/2006), published on 9 August 2019*.*

The pyrogenic load of the particles obtained is below 1 EU/mg and the microbial load of the particles obtained is below 300 CFU/mg.

After sterilizing the particles obtained with beta radiation equal to or higher than 25 kGy, the microbial load of the particles obtained is below 100 CFU/g and the pyrogenic load is below 0.05 EU/mg.

Example 2. Process for the purification of PLA thermoplastic polymeric particles.

In this example, it is desired to purify polymeric particles of Resomer^{®} brand PLA.

Before starting, a sample of the commercial polymeric particles to be purified is analyzed under a microscope to assess the degree of visible non-polymeric (extrinsic) particles to be removed. Thus, fibers larger than 100 µm with a morphology very different from the PLA particles that are intended to be purified were observed under microscope. The image showed a clear indication that the PLA particles acquired (of pharmaceutical quality) are not really suitable for parenteral use and therefore require an additional purification process.

After observing the morphologically different particles, the researchers decided to analyse them by means of FTIR spectroscopy, where they observed that most of them are cellulose fibres. Therefore, the researchers proceeded with the dry purification process object of the present invention.

It should be noted that the purification process begins with an extrusion stage that takes place inside a reactor and that before starting the purification process, all equipment and materials to be used must be clean and sterile. For this purpose the equipment is previously sterilised with nebulised or vaporised hydrogen peroxide or with a mixture of hydrogen peroxide and peracetic acid.

To begin the purification process, the polymer in the form of particles is incorporated into the reactor. Once the polymeric particles are introduced into the reactor, the reactor is closed and pressurized with an inert gas such as nitrogen. With the partially molten PLA particles, the reactor is stirred with the help of a propeller, said stirring continuing until a temperature of at least 5°C ± 10% above the melting temperature (Tm) of the polymer is reached, which in each case will be established by the nature and composition thereof, and which generally lies between 50°C and 300°C, and more particularly between 50°C and 180°C. Once these conditions are reached, stirring stops and the reactor is depressurized. Maintaining the temperature, stirring is stopped and the reactor is pressurized with nitrogen. After this, the discharge valve is opened so that the molten PLA flows from the reactor through a filter with a pore size of 100 µm. This filtration removes contaminating particles that have not been melted in the polymeric mass, obtaining a purified PLA with a specific gravity identical to that of the non-purified polymer.

The polymeric mass is then cooled by a sterile air stream to a temperature of at least 4.5 to 5.5°C below Tg, and breaking down of the cooled polymer sample is subsequently started to obtain polymeric particles with a particle diameter ≥ 1 mm.

Finally, a sample of the particles is taken after the purification process and they are observed again under a microscope and subsequently analyzed, observing how the cellulose fibers disappear from the initial commercial sample, as seen in example 1.

### Example 3. Process for the purification of polymeric microparticles

For the present example, a sample of particles obtained after the process of examples 1 and 2 is taken and the following steps are carried out:
a) drying the particles under vacuum for a time of at least 10 hours at room temperature, and
b) milling or sieving the particles until obtaining microparticles with an average diameter between 100 and 150 µm.

The sieving or milling is carried out by means of an in-line blade system that allows obtaining, in dry, an optimal dispersion of particle sizes, namely:
- D10: between 25 - 55 µm;
- D50: between 120 -170 µm;
- D90: between 300 - 375 µm.

Finally, a sample of the particles is taken after the purification and micronization process (drying / milling and/or sieving) to be observed again under a microscope. Subsequently, they are analyzed, observing how the cellulose fibers disappear from the initial commercial sample, as seen in Figure 7.

### Comparative Examples:

Comparison of densities of products purified by prior art methods and non-purified products:
This example compares the bulk and tapped density of a commercial polymer (45 RESOMER^{®} RG 503 H GMP and 26 RESOMER^{®} RG 504 H GMP) purified with solvents, and another commercial polymer of identical characteristics (RESOMER^{®} Select 5050 DLG SE-Mill) that is non-purified.

| Polymer | Purification | Intrinsic Viscosity (IV) (dL/g) | ρ_{b} (g/cm³) | ρₜ (g/cm³) |
|---|---|---|---|---|
| RESOMER^{®} Select 5050 DLG SE-Mill | Non-purified | 0.45-0.60 | 0.64 | 0.84 |
| 45 RESOMER^{®} RG 503 H GMP | Solvent purified | 0.33-0.44 | 0.09±0.02 | 0.11 ± 0.02 |
| 26 RESOMER^{®} RG 504 H GMP | Solvent purified | 0.45-0.60 | 0.09±0.02 | 0.10± 0.01 |

As seen in the table, purification using solvents results in a very reduced bulk density and tapped density compared to the same non-purified product.

On the other hand, a polymer of similar characteristics purified by the method of the present invention results in a polymer with the same density characteristics as the starting product (example 1).

Residual amount of solvent in products of the prior art, purified by methods using solvents:

| | | |
|---|---|---|
| Polymer (Sigma-Aldrich) | Purification | Residual Solvents (GLC-HS) |
| Resomer^{®} RG 502 H | Solvent purified | 23 PPM TOLUENE |
| | | 0.01% ACETONE |
| | | 0.02% TOTAL |

As can be seen, the residual amount of solvents is much higher than that of polymeric particles obtained by the process of the invention (0.00% in Example 1).

## Claims

1. Process for dry purification of biodegradable thermoplastic polymeric particles for medical and/or pharmaceutical use, which comprises the following steps:
a).- providing a biodegradable particulate thermoplastic polymer in a reactor;
b).- extruding the polymer at a temperature of 4.5 to 5.5°C above the melting temperature (Tm) of the selected polymer and with positive pressure greater than 0.1 bar in the presence of an inert gas;
c).- filtering the polymer through at least one filtration step with at least one filter of pore size from 5 to 300 µm;
d).- cooling the extruded polymer by means of a sterile air stream to a temperature below that of step b) until the polymer reaches a temperature of at least 4.5 to 5.5 °C below the glass transition temperature (Tg); and
e).- disintegrating the cooled polymer sample to obtain polymeric particles with a particle diameter ≥ 1 mm.

2. Process according to claim 1, wherein the filter of step c) has a pore size of 100 to 150 µm.

3. Process according to claim 2, **characterized in that** the filtration step c) is performed with a plurality of filters located in series, in parallel, in cascade or in any of its combinations.

4. Process according to any of claims 1 to 3 **characterized by** that between the extrusion step b) and the filtration step c) a drive pump of the extruded polymer is incorporated in order to promote filtering.

5. Process according to any of the preceding claims **characterized by** that, after step e), the process comprises a further drying step f) and a further milling and/or sieving step g) which is carried out by applying vacuum for a time of at least 10 hours at room temperature under sterile conditions, wherein particles with a diameter of ≤ 1 mm are obtained.

6. Process according to any of the preceding claims **characterized by** that, after step e), f) or g), the process comprises an additional sterilization step h) wherein the polymeric particles are subjected to a Beta Radiation dose which is equal to or greater than 25 kGy.

7. Process according to any of the preceding claims **characterized in that** the cooling step d) is carried out with sterile compressed air through a HEPA filter of pore size 0.22 µm.

8. Process according to any of the preceding claims **characterized in that** the cooling step d) is carried out with sterile inert gas.

9. Process according to any of the preceding claims **characterized in that** the disintegrating step e) is performed by a system of blades in line.

10. Process according to any of the preceding claims **characterized in that** it is carried out under sterile conditions.

11. Process according to any of the preceding claims, wherein the extrusion step b) is carried out in a reactor with agitation by blade or paddle, turbine, propeller, anchor, spiral or screw without end.

12. Process according to any of claims 1 to 11, wherein, prior to step a), the reactor is pressurized using an inert gas selected from the group consisting of nitrogen, argon, helium and compressed gas.

13. Process according to any of the preceding claims, wherein the filter used in the filtration step c) is a pharmaceutical grade filter such as 304 stainless steel or 316 stainless steel.

14. Process according to any of the preceding claims, wherein the polymeric particles of step a) are selected from the group consisting of particles of homopolymers and copolymers of lactic acid (L, D, DL), glycolic and/or ε-caprolactone such as polymer poly(L-lactic), poly(D-lactic), poly(DL-lactic), polyglycolic, poly(ε-caprolactone), copolymers of L-lactic/S-lactic acids, L-lactic/DL-lactic, L-lactic/glycolic, DL-lactic/glycolic or L-lactic/ε-caprolactone, PC, PE, PEEK, PEI, PES, POM, PP, PPS, PPSU, PS, PSU, PTFE or UHMWPE.

15. Process according to any of the preceding claims **characterized by** comprising the following additional steps:
f) vacuum drying the particles for at least 10 hours at room temperature; and
g) milling or sieving the particles to microparticles with an average diameter between 100 and 150 µm.

16. Process according to the preceding claim **characterized in that** the polymeric particles are PLGA or PLA microparticles.

17. Process according to any of the preceding claims **characterized in that** microparticles with the following distribution are obtained:
- D10: between 25 - 55 µm;
- D50: between 120 -170 µm; and
- D90: between 300 - 375 µm.

18. Process according to any of the preceding claims **characterized in that** the biodegradable thermoplastic polymer provided in step a) has not been previously purified or has not been previously purified with a method using solvents.

19. Polymeric particle obtained according to any one of preceding claims 1 to 18 **characterized in that** it is sterile suitable for parenteral formulations.

20. Polymeric particle obtained according to any one of preceding claims 1 to 18 **characterized in that** it is sterile suitable for manufacturing implantable medical devices.

21. Biodegradable thermoplastic polymeric particles **characterized by** having a bulk density of 0.10 to 9.0 g/cm³, a compacted density of 0.13 to 12.0 g/cm³, a residual solvent quantity of 0.00 %, a pyrogenic load below 1 EU/mg and a microbial load below 300 CFU/mg, with a polymer purity level of at least 95%, preferably at least 97% and more preferably 99%, and a particle size distribution defined as follows:
- D10: between 25 - 55 µm;
- D50: between 120 -170 µm; and
- D90: between 300 - 375 µm.

22. The biodegradable thermoplastic polymeric particles according to claim 21, wherein the particles are selected from the group consisting of particles of homopolymers and copolymers of lactic acid (L, D, DL), glycolic and/or ε-caprolactone such as polymer poly(L-lactic), poly(D-lactic), poly(DL-lactic), polyglycolic, poly(ε-caprolactone), copolymers of L-lactic/S-lactic acids, L-lactic/DL-lactic, L-lactic/glycolic, DL-lactic/glycolic or L-lactic/ε-caprolactone, PC, PE, PEEK, PEI, PES, POM, PP, PPS, PPSU, PS, PSU, PTFE or UHMWPE.

23. Use of the biodegradable thermoplastic polymeric particles according to any one of claims 19 to 22 to manufacture medicaments for parenteral administration and/or implantable medical devices.

24. Biodegradable thermoplastic polymeric particles according to any one of claims 19 to 22 for use as medicaments for parenteral administration and/or implantable medical devices.

25. Method of treating a disease which comprises administering the polymeric particles according to claims 19 to 22 and/or implanting the medical devices obtained with the polymeric particles according to claims 19 to 22.
